# EUROPÄISCHE PATENTSCHRIFT

(11) **EP 1 259 800 B1**
(45) Veröffentlichungstag und Bekanntmachung des Hinweises auf die Patenterteilung: **21.06.2006**
(21) Anmeldenummer: 01916899.6
(22) Anmeldetag: 28.02.2001
(51) Int. Cl.: G01N 27/327, C12Q 1/00, G01N 27/403

(54) **ENZYMATISCH-ELEKTROCHEMISCHE MESSEINRICHTUNG**
ENZYMATIC-ELECTROCHEMICAL MEASURING DEVICE
DISPOSITIF DE MESURE ENZYMATIQUE ELECTROCHIMIQUE

(30) Priorität: 28.02.2000 DE 10009467
(43) Veröffentlichungstag der Anmeldung: 27.11.2002
(73) Patentinhaber: Ernst-Moritz-Arndt-Universität Greifswald, 17487 Greifswald (DE)
(72) Erfinder: ABEL, Peter, 17495 Züssow (DE); VON WOEDTKE, Thomas, 18519 Horst (DE); SCHWOCK, Alexander, 18516 Griebenow (DE)
(74) Vertreter: Neumann, Günter
(86) Internationale Anmeldenummer: PCT/DE2001/000824
(87) Internationale Veröffentlichungsnummer: WO 2001/064938

(56) Entgegenhaltungen:
- US-A- 3 979 274
- US-A- 5 312 590
- US-A- 5 863 400
- MCGRATH M J ET AL: "The use of differential measurements with a glucose biosensor for interference compensation during glucose determinations by flow injection analysis." BIOSENS. BIOELECTRON., Bd. 10, Nr. 9/10, 1995, Seiten 937-943, XP001051282 ISSN: 0015-3230
- LU B ET AL: "Development of an amperometric immunosensor based on flow injection analysis for the detection of red blood cells." ANALYTICA CHIMICA ACTA, Bd. 340, Nr. 1-3, 1997, Seiten 175-180, XP001058485
- DATABASE WPI Section Ch, Week 198541 Derwent Publications Ltd., London, GB; Class A89, AN 1985-249614 XP002187849 & CS 8 302 656 A (ZENT DIABETES KATSC), 13. Juni 1985 (1985-06-13) in der Anmeldung erwähnt
- WANG J ET AL: "Needle-type dual microsensor for the simultaneous monitoring of glucose and insulin" ANALYTICAL CHEMISTRY, Bd. 73, Nr. 4, 15. Februar 2001 (2001-02-15), Seiten 844-847, XP002187848

## Beschreibung

Die Erfindung betrifft eine enzymatisch-elektrochemische Meßeinrichtung mit Clark-Elektroden mit einem breiten Anwendungsgebiet über die medizinische Diagnostik hinaus.

Zur Kontrolle von Stoffwechselsituationen, insbesondere bei Diabetikern, ist die regelmäßige Kontrolle der zirkulierenden Glucosekonzentration erforderlich. Das gilt nicht nur für Insulin injizierende Typ1 - Diabetiker, sondern auch für Typ2 - Diabetiker, wobei letztere etwa 85 % der Zuckerkranken ausmachen. Weltweit wird die Zahl der Diabetiker im Jahr 2010 voraussichtlich bei 221 Millionen Betroffenen liegen (Hauner, H.; Dtsch Med Wochenschr 1998; 123; 777-82; Pharmaco Economics 1995; 8 (Suppl. I) 28-71; Ammon, H. P. T.; Deutsche Apoteker Zeitung; 1999; 139 Jahrgang Nr. 30).

Gemessen an der hohen Anzahl an Diabetes Erkrankter kann die einzige routinemäßig eingeführte Methode der Blutzuckermessung nach Penetration der Haut die Forderung und den Wunsch nach regelmäßiger Stoffwechselkontrolle nicht erfüllen. Das ist sowohl in der vor jeder Messung erforderlichen Überwindung der psychischen Schranke zur Verletzung der Haut als auch im methodisch bedingten materiellen Aufwand für die Realisierung jeder Messung ausschließlich unter Verwendung von Einweg-Materialien begründet.

Bei der überwiegenden Anzahl der Betroffenen ist eine Blutzuckermessung auch nicht indiziert, sondern eine Kontrolle der Anwesenheit von Glucose im Urin ausreichend. Glucose im Urin ist dann in detektierbarer Konzentration vorhanden, wenn durch Irritationen des Stoffwechsels die Nierenschwelle überschritten ist, so dass zusätzlich zur physiologischen Verwertung Glucose als Harnzucker über die Nieren ausgeschieden wird.

Eine bekannte Methode zur Bestimmung des Harnzuckers beruht auf der Eigenschaft der Glucose, polarisiertes Licht rechts zu drehen. Diese Eigenschaft wird im sogenannten Polarimeter genutzt, wobei der Ablenkungswinkel des Lichtes aus der Polarisationsebene das Maß für die aktuelle Glucosekonzentration der zu vermessenden Probe ist (Pöge, A.W. Z. med. Labortechnik 17 (1976) 59 - 78).

Insbesondere für screening-Maßnahmen zu Präventionszwecken werden auch Hamzuckerteststreifen angewendet, die zumeist auf enzymatisch-chemischer Basis eine glucoseabhängige Farbreaktion generieren. Diese Teststreifen haben den Nachteil großer Ungenauigkeit und dadurch der Unzuverlässigkeit des Messergebnisses und sind nur von farbtüchtigen Menschen auswertbar. Gerade aber bei Diabetikern, insbesondere Älteren des Typs 2, ist bei einer erheblichen Anzahl mit Farbuntüchtigkeit, zumindest aber mit Farbunsicherheit zu rechnen.

Während das Polarimeter als Laborgerät zur Anwendung in der klinischen Chemie etabliert ist, sind Harnzuckerteststreifen für eine zuverlässige Aussage über die Anwesenheit von Glucose und deren Konzentration in der täglichen Stoffwechselkontrolle als Routinemethode nicht geeignet.

Forschung und Entwicklung konzentrierten sich daher zunehmend auf enzymatisch-elektrochemische Messeinrichtungen und deren Anwendung für die Glucosebestimmung.

Der dem klassischen Clark-Sauerstoffsensor entsprechende elektrochemische Grundsensor des enzymatisch-elektrochemischen Biosensors ist bekannt und in der Literatur vielfach beschrieben.

So wird in der DD 227 029 A3 eine Anordnung des enzymatisch-elektrochemischen Biosensors zur Bestimmung von Analyten in Flüssigkeiten offenbart.
Gemäß dieser zur Glucosemessung bestimmten technischen Lösung besteht die Enzymelektrode aus einer Sandwich-Membrananordnung, die eine hydrophobe und eine hydrophile Membran mit einem dazwischen gelagerten Enzym aufweist. Diese Anordnung nutzt die modifizierte klassische Clark-Elektrode ausschließlich zur Bestimmung des infolge einer enzymatischen Oxidation des zu detektierenden Analyten unter Verbrauch von Sauerstoff generierten Wasserstoffperoxids, welches an der Anode bei einem Potential von 600 mV bis 700 mV elektrochemisch oxidiert wird, als Maß für die Analytkonzentration. Diese Anordnung hat den Nachteil, dass andere elektrochemisch aktive Substanzen aus der Probe, die ebenfalls durch das Membransystem diffundieren können, in die Reaktionsschicht des Biosensors gelangen und ebenso wie das analytspezifisch generierte Wasserstoffperoxid an der Arbeitselektrode oxidiert werden können und somit als interferierende Substanzen zu einer Verfälschung des Meßsignals führen.

Des weiteren können derartige an die Arbeitselektrode gelangende Substanzen diese unspezifisch in ihren elektrochemischen Eigenschaften beeinflussen und damit zu einer Elektrodenvergiftung und -alterung führen.

Die interferierende Wirkung unspezifischer Analyte kann durch die Wahl eines geeigneten Elektrodenpotentials zwischen Arbeits- und Referenzelektrode im Zusammenhang mit einem Mediator, der anstelle des Sauerstoffs als Elektronenakzeptor fungiert, verringert werden, wobei eine Absenkung des Elektrodenpotentials bis etwa 300 mV möglich ist. Der generelle Zutritt von Fremdmolekülen zum Elektrodensystem bleibt jedoch auch bei dieser Methode unvermeidbar.

Die genannten Nachteile führen dazu, dass das Meßergebnis durch interferierende Substanzen bzw. durch Elektrodenvergiftung oder -alterung derart stark beeinflußt werden kann, dass die gewünschte Aussage zur Anwesenheit des eigentlich zu detektierenden Analyten nicht sicher, stabil und reproduzierbar gemacht werden kann.

Aufgabe der Erfindung ist es daher, eine wiederverwendbare auf enzymatischelektrochemischer Basis arbeitende verbesserte Vorrichtung bereitzustellen, mit der es möglich ist, die Anwesenheit und Konzentration von Analyten in wässrigen Lösungen auf einfachere Weise stabil und reproduzierbar zu bestimmen und anzuzeigen. Insbesondere liegt der Erfindung die Aufgabe zugrunde, eine Vorrichtung bereitzustellen, mit der es unter den Bedingungen des täglichen Lebens auch für ältere Menschen möglich ist, durch Routineuntersuchungen die Anwesenheit bzw. Konzentration von Glucose im Urin zu messen und auf geeignete Weise anzuzeigen und/oder zu signalisieren.

Erfindungsgemäß wird die Aufgabe durch eine enzymatisch-elektrochemische Messeinrichtung mit den Merkmalen des Anspruches 1 gelöst. Vorteilhafte Ausgestaltungen der erfindungsgemäßen Messeinrichtung ergeben sich aus den Merkmalen der Unteransprüche 2 bis 8. Die erfindungsgemäße Verwendung der Messvorrichtung ergibt sich aus den Merkmalen der Ansprüche 9 und 10.

Die erfindungsgemäße Messeinrichtung hat den Vorzug, dass sie vielseitig zur Bestimmung, Kontrolle und Überwachung der Anwesenheit und Konzentration unterschiedlicher Analyten in der medizinischen Diagnostik sowie in der Lebensmittel- und biotechnologischen Industrie eingesetzt werden kann. Sie basiert auf einem als Doppelsensor ausgebildeten enzymatisch-elektrochemischen Biosensor nach dem per se bekannten Prinzip einer Clark-Sauerstoffelektrode in Zusammenhang mit einem für die Aufnahme des dualen Biosensors und eine ausreichende Probemenge geeigneten Gefäßes.

Zur Glucosebestimmung ist das Probegefäß so als Überlaufgefäß ausgebildet, dass es eine ausreichende Menge Urin am sensitiven Bereich des Biosensors zurückhält. Das den dualen enzymatisch-elektrochemischen Biosensor aufnehmende Gefäß ist in der Weise konstruiert, dass es eine definierte und für die jeweilige Meßaufgabe bestimmte Teilmenge des Urins während des Ausscheidungsvorgangs aufnehmen kann. Es ist konstruktiv dafür gesorgt, dass der Teil des Gefäßes, in den der duale enzymatisch-elektrochemische Biosensor für Glucose eingebracht ist, bis zur Überlaufgrenze gefüllt wird, während überschüssige Mengen frei abfließen können.

Im folgenden sollen die Funktionsweise der erfindungsgemäßen Messvorrichtung und deren Vorzüge im Verhältnis zum Stand der Technik am Beispiel der Bestimmung von Glucose im Urin näher erläutert werden, ohne die Erfindung in ihrem Umfang zu beschränken.

Aus dem Stand der Technik (DD 227 029 A3) ist bekannt, dass physikalisch gelöster Sauerstoff eine der klassischen Clark-Elektrode vorgelagerte nur gasdurchlässige hydrophobe Membran passiert und an einer Arbeitselektrode elektrochemisch reduziert wird. Dabei werden je Sauerstoffmolekül zwei Elektronen frei, die einen Strom generieren, der die aktuelle Konzentration des physikalisch gelösten Sauerstoffs, den Sauerstoffpartialdruck, repräsentiert.

Im Falle des ebenfalls bekannten enzymatisch-elektrochemischen Biosensors für Glucose ist der hydrophoben Membran eine enzymatische Schicht vorgelagert, die vorzugsweise das Enzym Glucoseoxidase enthält und die ihrerseits durch eine für gelöste Ionen und Moleküle durchlässige, hydrophile Membran abgedeckt ist. Diffundiert nun aus der Lösung, in der sich der Sensor befindet, ein Analyt ein, dessen Oxidation durch das vorhandene Enzym katalysiert wird, so wird auf Grund des Sauerstoffverbrauchs bei dieser Reaktion der Sauerstoffpartialdruck an der Arbeitselektrode verringert. Das heißt, dass der in diesem Fall gemessene Sauerstoffpartialdruck um den Betrag vermindert wird, der durch die enzymatische Reaktion verbraucht wird. Auf diese Weise ist die Differenz zwischen dem ursprünglichen Sauerstoffpartialdruck in der Lösung und dem um den jeweiligen Betrag verminderten Sauerstoffpartialdruck an der Arbeitselektrode ein Maß für die Konzentration des zu detektierenden Analyten. Die Bewertung dieser Partialdruckdifferenz setzt allerdings voraus, dass der ursprüngliche Sauerstoffpartialdruck im zu analysierenden Medium konstant und bekannt ist. Diese Voraussetzung ist jedoch in biologischen Lösungen nicht gegeben. An dieser Stelle setzt die erfindungsgemäße Lösung an.

Um dennoch die Messung des Sauerstoffverbrauchs als Indikator für aktuell vorhandene Glucose in der zu untersuchenden Probe nutzen zu können, ist der bekannte elektrochemische Grundsensor erfindungsgemäß in der Weise weiterentwickelt und ausgelegt worden, dass zu dem klassischen Clark-ElektrodenSystem eine weiterer Sensor hinzugefügt wurde, dessen hydrophobe Membranbedeckung der Arbeitselektrode nicht mit dem für die Oxidation des zu detektierenden Analyten zutreffenden Enzym beschichtet ist. An dieser Arbeitselektrode wird der Sauerstoff entsprechend seiner aktuellen Konzentration in der zu analysierenden Probe reduziert, da kein durch eine enzymatische Reaktion bedingter Sauerstoffverbrauch stattfindet. Die Signale der Arbeitselektroden des auf diese Weise gebildeten dualen Sensors werden erfindungsgemäß einem Differenzverstärker zugeführt, in dem die für den Fall der Anwesenheit des Analyten Glucose aktuelle Signaldifferenz gebildet wird. Die weitere Verarbeitung dieses spezifischen Meßsignals geschieht optional in einer vom Anwender bevorzugten Weise.

Mit der erfindungsgemäßen Lösung verbindet sich der besondere Vorzug, dass ein vollständiger, jedoch selektiver Ausschluß von die Messung beeinflussenden Substanzen von der Arbeitselektrode einerseits, ein ebenso vollständiger und selektiver Zugang des zu detektierenden Analyten oder eines Produktes einer spezifischen Detektionsreaktion an die Arbeitselektrode andererseits, gewährleistet ist. Interferenzen und eine Elektrodenvergiftung werden dadurch ausgeschlossen.

Das den dualen enzymatisch-elektrochemischen Biosensor sowie eine für die entsprechende Meßaufgabe ausreichende Urinmenge aufnehmende Überlaufgefäß kann erfindungsgemäß sowohl als portable als auch im WC-Becken fixierte Einrichtung ausgelegt werden.
Auf diese Weise ist die industriell herstell- und einfach handhabbare erfindungsgemäße Lösung zur Harnzuckerbestimmung sowohl stationär als auch ambulant geeignet und somit auch durch ältere betroffene Menschen unter häuslichen Bedingungen im täglichen Leben anwendbar. Die Anzahl der möglichen Messungen wird ausschließlich durch die Lebensdauer des Biosensors begrenzt.

Wird das Enzym Glucoseoxidase durch andere ebenfalls sauerstoffverbrauchende Reaktionspartner substituiert, können auch andere Analyte in Medien unbekannter Zusammensetzung bestimmt werden. So besteht zum Beispiel die Möglichkeit, durch Verwendung von Lactatoxidase als Enzym die Konzentration von Lactat im Zuckerrübenrohsaft oder anderen Flüssigkeiten zu messen und anzuzeigen.

## Patentansprüche

1. Enzymatisch-elektrochemische Meßeinrichtung basierend auf einer Clark-Elektrode bestehend aus einer Ag/AgCl-Referenzelektrode als Anode und einer elektrochemisch aktiven Arbeitselektrode, die mit einer Elektrolytschicht sowie einer hydrophoben Membran und einem Enzym als Sensor ausgebildet sind, **dadurch gekennzeichnet, dass** mindestens zwei der Sensoren als duale Sensoren angeordnet sind, die Elektroden beider Sensoren mit einer Elektrolytschicht und einer Membran hydrophoben Charakters bedeckt und gleichmäßig benetzt sind, die hydrophobe Membran ein vorwiegend gasdurchlässiges an sich bekanntes Polymer ist, auf dem nur bei der Arbeitselektrode eines der Sensoren ein Enzym immobilisiert ist, während die hydrophobe Membran des anderen Sensors enzymfrei bleibt.

2. Enzymatisch-elektrochemische Messeinrichtung nach Anspruch 1, **dadurch gekennzeichnet, dass** die elektrochemische Arbeitselektrode eine Gold-, Platin-, Kohlenstoffelektrode oder eine Elektrode aus einem sauerstoffaktiven Katalysator, vorzugsweise auf Ruthenium-Selen oder Eisen-Basis.

3. Enzymatisch-elektrochemische Messeinrichtung nach Anspruch 1 oder 2, **dadurch gekennzeichnet, dass** das Enzym ein sauerstoffverbrauchendes Enzym, eine Oxidase ist, vorzugsweise Glucose-Oxidase, Lactat-Oxidase, Alkohol-Oxidase, Sulfitoxidase, Urease oder eine Peroxidase ist.

4. Enzymatisch-elektrochemische Meßeinrichtung nach einem der Ansprüche 1 bis 3, **dadurch gekennzeichnet, dass** die enzymbehaftete Elektrode durch Kopplung verschiedener Enzyme als Bi- oder Multienzymelektrode ausgebildet ist, vorzugsweise durch Kopplung von Enzymen aus der Gruppe der O-xidasen und von Enzymen der Gruppe der Peroxidasen.

5. Enzymatisch-elektrochemische Meßeinrichtung nach einem der Ansprüche 1 bis 4, **dadurch gekennzeichnet, dass** die enzymbeschichtete Membran mit einer hydrophilen Membran bedeckt ist.

6. Enzymatisch-elektrochemische Meßeinrichtung nach einem der Ansprüche 1 bis 5, **dadurch gekennzeichnet, dass** der nicht mit Enzym versehene Sensor den ursprünglich ungestörten Sauerstoffpartialdruck der Probelösung und der enzymbeschichtete Sensor den durch den infolge der enzymatischen Reaktion verringerten Sauerstoffpartialdruck misst.

7. Enzymatisch-elektrochemische Meßeinrichtung nach einem der Ansprüche 1 bis 6, **dadurch gekennzeichnet, dass** die Signale der beiden Sensoren einem Differenzverstärker zugeführt werden und der Differenzverstärker die Differenz beider Elektrodensignale als qualitativen und quantitativen Indikator für die An- bzw. Abwesenheit des zu detektierenden Analyten beziehungsweise dessen Konzentration bildet und diese Werte optional optisch oder akustisch, analog oder digital ausgegeben werden.

8. Enzymatisch-elektrochemische Meßeinrichtung nach einem der Ansprüche 1 bis 7, **dadurch gekennzeichnet, dass** die Sensoren in einem Überlaufgefäß angeordnet sind und das Überlaufgefäß so ausgelegt ist, dass bei jedem Meßvorgang ein für die Messung ausreichender Füllstand gewährleistet ist.

9. Verwendung der enzymatisch-elektrochemischen Meßeinrichtung nach einem der Ansprüche 1 bis 8 zur Bestimmung, Kontrolle und Überwachung der Anwesenheit und Konzentration von Glucose im Urin sowie zur Bestimmung, Kontrolle und Überwachung der Anwesenheit und Konzentration von Glucose, Lactat, Harnsäure, Sulfit, Maltose, Saccharose, Glutamat, Fructose, Pyruvat, Phenolen, Glutamin, Galactose und Lysin.

10. Verwendung nach Anspruch 9, **dadurch gekennzeichnet, dass** zur Bestimmung, Kontrolle und Überwachung der Anwesenheit und Konzentration von Glucose im Urin die enzymatisch-elektrochemische Meßeinrichtung in WC-Becken und/oder Urinalen stationär angeordnet oder konstruktiv so gestaltet ist, dass sie mobil anwendbar ist.

## Claims

1. Enzymatic-electrochemical measuring device, based on a Clark electrode, comprising an Ag/AgCl reference electrode as the anode and an electrochemically active working electrode being configured from a layer of electrolyte, a hydrophobic membrane and an enzyme as a sensor, **characterized by** at least two of the sensors are arranged as dual sensors with an electrolytic layer and a membrane with hydrophobic properties and wetted in a uniform manner, the hydrophobic membrane is a predominantly gas-permeable polymer on which, in the case of the working electrode an enzyme is immobilized, whereas the hydrophobic membrane of the other sensor is devoid of enzymes.

2. Enzymatic-electrochemical measuring device in accordance with claim 1, **characterized by**, the electrochemical working electrode being a gold, platinum, carbon electrode or an electrode from an oxygen-activated catalyst, preferably on ruthenium-selenium or iron basis.

3. Enzymatic-electrochemical measuring device in accordance with claim 1 or 2, **characterized by** the enzyme being an oxygen-consuming enzyme, oxidase, preferably glucose oxidase, lactate oxidase, alcohol oxidase, sulfite oxidase, urease or a peroxidase.

4. Enzymatic-electrochemical measuring device in accordance with one of the claims 1 to 3, **characterized by** the enzyme-covered electrode being formed by coupling of various enzymes as bi- or multi-enzymatic electrodes, preferably by coupling enzymes from the group of oxidase and from enzymes of the group of perioxidases.

5. Enzymatic-electrochemical measuring device in accordance with one of the claims 1 to 4, **characterized by** the enzyme-coated membrane is covered with a hydrophilic membrane.

6. Enzymatic-electrochemical measuring device in accordance with one of the claims 1 to 5, **characterized by** the sensor which is not covered with enzymes measures the originally undisturbed partial oxygen pressure of the sample solution and the enzyme-coated sensor measures the partial oxygen pressure reduced by the enzymatic reaction.

7. Enzymatic-electrochemical measuring device in accordance with one of the claims 1 to 6, **characterized by** the signals of the two sensors passing through a difference amplifier and the difference amplifier establishes the difference of the two electrode signals as qualitative and quantitative indicators for the presence or lack of the analytes to be detected or their concentration and displays these values either optically or acoustically, analog or digitally.

8. Enzymatic-electrochemical measuring device in accordance with one of the claims 1 to 7, **characterized by** the sensors being placed in an overflow vessel and the overflow vessel is such that a sufficient level for the measurement is guaranteed for every measurement.

9. Use of the enzymatic-electrochemical measuring device in accordance with one of the claims 1 to 8 for the determination, control and monitoring of the presence and concentration of glucose in urine and the determination, control and monitoring of glucose, lactate, uric acid, sulfite, maltose, saccharine, glutamate, fructose, pyruvate, phenolene, glutamine, galactose and lysine.

10. Use in accordance with claim 9, **characterized by** the enzymatic-electrochemical measuring device is attached permanently to toilets and urinals and determination, control and monitoring of the presence and concentration of glucose in urine or constructed so that they can be used portably.

## Revendications

1. Dispositif de mesure électrochimique enzymatique basé sur une électrode de Clark, se composant d'une électrode de référence Ag/AgCl considérée comme anode et d'une électrode de travail à action électrochimique, lesdites électrodes se composant d'une couche d'électrolyte ainsi que d'une membrane hydrophobe et d'une enzyme en tant que capteur, **caractérisé en ce qu'**au moins deux des capteurs sont arrangés sous forme de bicapteurs, les électrodes des deux capteurs sont recouvertes d'une couche d'électrolyte et d'une membrane à caractère hydrophobe en étant imprégnées uniformément, la membrane hydrophobe est un polymère connu en soi, principalement perméable au gaz, sur lequel est immobilisée une enzyme seulement dans le cas de l'électrode de travail de l'un des capteurs alors que la membrane hydrophobe de l'autre capteur est dépourvue d'enzyme.

2. Dispositif de mesure électrochimique enzymatique selon la revendication 1, **caractérisé en ce que** l'électrode de travail électrochimique est une électrode en or, en platine et en carbone ou une électrode composée d'un catalyseur à l'oxygène actif, de préférence à base de ruthénium-sélénium ou de fer.

3. Dispositif de mesure électrochimique enzymatique selon la revendication 1 ou 2, **caractérisé en ce que** l'enzyme est une enzyme consommatrice d'oxygène, une oxydase, de préférence une glucose oxydase, lactate oxydase, alcool oxydase, sulfite oxydase, une uréase ou une péroxydase.

4. Dispositif de mesure électrochimique enzymatique selon l'une quelconque des revendications 1 à 3, **caractérisé en ce que** l'électrode chargée d'enzyme est réalisée sous forme d'électrode bienzymatique ou multienzymatique grâce à l'association de diverses enzymes, de préférence grâce à l'association d'enzymes du groupe des oxydases et d'enzymes du groupe des péroxydases.

5. Dispositif de mesure électrochimique enzymatique selon l'une quelconque des revendications 1 à 4, **caractérisé en ce que** la membrane enduite d'enzyme est recouverte d'une membrane hydrophile.

6. Dispositif de mesure électrochimique enzymatique selon l'une quelconque des revendications 1 à 5, **caractérisé en ce que** le capteur dépourvu d'enzyme mesure la pression partielle de l'oxygène intacte à l'origine de la solution échantillon, et le capteur enduit d'enzyme la pression partielle de l'oxygène réduite suite à la réaction enzymatique.

7. Dispositif de mesure électrochimique enzymatique selon l'une quelconque des revendications 1 à 6, **caractérisé en ce que** les signaux des deux capteurs sont amenés à un amplificateur de différences, et l'amplificateur de différences forme la différence des deux signaux d'électrode sous forme d'indicateur de qualité et de quantité pour la présence ou l'absence de l'analyte à détecter ou de sa concentration, et ces valeurs sont émises au choix par des moyens optiques, acoustiques, analogiques ou numériques.

8. Dispositif de mesure électrochimique enzymatique selon l'une quelconque des revendications 1 à 7, **caractérisé en ce que** les capteurs sont disposés dans un récipient de trop-plein, et le récipient de trop-plein est dimensionné de sorte à assurer à chaque mesure un niveau de remplissage suffisant à la mesure.

9. Utilisation du dispositif de mesure électrochimique enzymatique selon l'une quelconque des revendications 1 à 8 en vue de déterminer, contrôler et surveiller la présence et la concentration de glucose dans l'urine ainsi que de déterminer, contrôler et surveiller la présence et la concentration de glucose, lactate, acide urique, sulfite, maltose, saccharose, glutamate, fructose, pyruvate, phénols, glutamine, galactose et lysine.

10. Utilisation selon la revendication 9, **caractérisée en ce qu'**en vue de déterminer, contrôler et surveiller la présence et la concentration de glucose dans l'urine, le dispositif de mesure électrochimique enzymatique est disposé fixement dans les cuvettes de WC et/ou les urinoirs ou est construit de sorte à pouvoir être utilisé en dispositif mobile .
